(19) Europäisches Patentamt
European Patent Office
Office européen des brevets

(11) Veröffentlichungsnummer : **0 362 654 B1**

(12) **EUROPÄISCHE PATENTSCHRIFT**

(45) Veröffentlichungstag der Patentschrift :
**11.11.92 Patentblatt 92/46**

(51) Int. Cl.$^5$ : **C07C 275/60,** C09D 175/00

(21) Anmeldenummer : **89117642.2**

(22) Anmeldetag : **25.09.89**

(54) **Verfahren zur Herstellung von diblockierten Triisocyanatverbindungen.**

(30) Priorität : **29.09.88 AT 2401/88**

(43) Veröffentlichungstag der Anmeldung :
**11.04.90 Patentblatt 90/15**

(45) Bekanntmachung des Hinweises auf die Patenterteilung :
**11.11.92 Patentblatt 92/46**

(84) Benannte Vertragsstaaten :
**BE DE ES FR GB IT NL SE**

(56) Entgegenhaltungen :
**EP-A- 0 000 194**
**DE-B- 2 009 179**

(56) Entgegenhaltungen :
**ANGEWANDTE MAKROMOLEKULARE CHEMIE, Band 141, Juni 1986, Seiten 173-183, Basel, CH; D. WENDISCH et al:"Kernresonanzspektroskopische Beiträge zur Struktur und Stereochemie von (cyclo)aliphatischen Isocyanaten und deren Folgeprodukten"**

(73) Patentinhaber : **Vianova Kunstharz Aktiengesellschaft**
**A-8402 Werndorf (AT)**

(72) Erfinder : **Paar, Willibald, Dr.**
**Schanzelgasse 19**
**A-8010 Graz (AT)**
Erfinder : **Gmoser, Johann**
**Radegunderstrasse 30**
**A-8045 Graz (AT)**

**EP 0 362 654 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von diblockierten Triisocyanatverbindungen der allgemeinen Formel

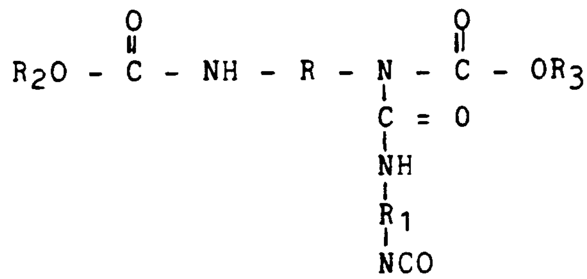

wobei

R ein von einem Diisocyanat stammender aliphatischer, cycloaliphatischer oder aromatischer Rest,

$R_1$ ein von einem Diisocyanat, dessen NCO-Gruppen überwiegend verschiedene Reaktivität aufweisen, stammender aliphatischer, cycloaliphatischer oder aromatischer Rest ist

und

$R_2$ und $R_3$ gleiche oder verschiedene Reste von NCO-Blokkierungsmitteln darstellen.

Es ist bekannt, Diisocyanate mit geeigneten Blockierungsmitteln vollständig oder halbseitig zu blockieren. Die Bildung der Monoisocyanatverbindung, d.h. die Halbblockierung im nichtstatistischen Ausmaß ist nur bei Diisocyanaten möglich, deren NCO-Gruppen unterschiedliche Reaktivität gegenüber dem Blockierungsmittel aufweisen. Bei der partiellen Blockierung von Triisocyanatverbindungen, wie z.B. den bekannten, aus 1 Mol Trimethylolpropan und 3 Mol Diisocyanat hergestellten Prepolymeren, kann nur eine statistische Verteilung der verschiedenen Blockierunsgrade erwartet werden.

Die erwähnten halbblockierten Diisocyanate werden, wie in vielen Literaturstellen beschrieben, zur Einführung von reaktionsfähigen Gruppen in Polymere benutzt, die als selbstvernetzende, wärmehärtende Lackbindemittel, besonders in wasserverdünnbaren Elektrotauchlacken, Verwendung finden.

Da bei Verwendung der üblichen halbblockierten Diisocyanate nur jeweils eine vernetzende Gruppe eingeführt wird, ist auf diese Weise eine ausreichende Vernetzung der Polymeren kaum zu erzielen.

Es besteht daher der Wunsch nach mono-NCO-funktionellen Verbindungen, die zwei weitere blockierte Isocyanatfunktionen aufweisen.

Es wurde nun gefunden, daß solche Verbindungen in einfacher Weise durch Allophanatbildung unter Verwendung blockierter und nichtblockierter Diisocyanate erhalten werden können.

Die vorliegende Erfindung betrifft demgemäß ein Verfahren zur Herstellung von diblockierten Triisocyanatverbindungen, welches dadurch gekennzeichnet ist, daß man ein Mol eines vollblockierten Diisocyanates mit einem Mol eines nichtblockierten Diisocyanates, dessen NCO-Gruppen überwiegend unterschiedliche Reaktivität aufweisen, bei 70 bis 120°C, gegebenenfalls in Gegenwart eines Katalysators, bis zu einem der Monoisocyanatverbindung entsprechenden NCO-Wert reagiert, wobei die maximale Reaktionstemperatur so gewählt wird, daß noch keine wesentliche Reaktion der freien NCO-Gruppe und/oder noch keine wesentliche Abspaltung des Blockierungsmittels erfolgt.

Die Erfindung betrifft weiterhin diblockierte Triisocyanatverbindungen der allgemeinen Formel

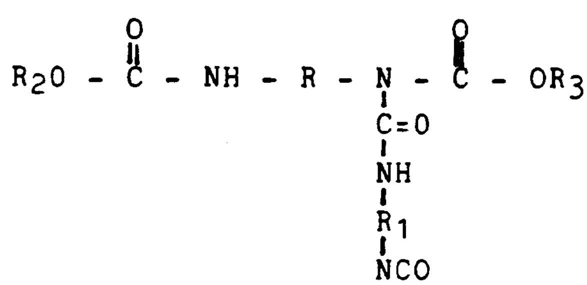

wobei

R ein von einem Diisocyanat stammender aliphatischer, cycloaliphatischer oder aromatischer Rest,

$R_1$ ein von einem Diisocyanat, dessen NCO-Gruppen überwiegend verschiedene Reaktivität aufweisen, stammender aliphatischer, cycloaliphatischer oder aromatischer Rest ist

und

$R_2$ und $R_3$ gleiche oder verschiedene Reste von NCO-Blokkierungsmitteln darstellen.

Weiters betrifft die Erfindung die Verwendung der erfindungsgemäß hergestellten diblockierten Triisocyanate als Vernetzungskomponente für Hydroxylgruppen und/oder primäre und/oder sekundäre Aminogruppen aufweisende Lackbindemittel. Eine bevorzugte Verwendung betrifft die Einführung blockierter Urethanvernetzungsgruppen in selbstvernetzende Lackbindemittel. Gegebenenfalls kann auf diesem Wege auch die Einführung protonierbarer Gruppen erfolgen.

Es war überraschend und nicht vorherzusehen, daß nach dem beanspruchten Verfahren weitgehend einheitliche Produkte hergestellt werden können. Es hat sich gezeigt, daß die Reaktivität der zweiten NCO-Gruppe so wesentlich geringer ist, daß bei richtiger Reaktionsführung der NCO-Wert der Monoisocyanatverbindung auch bei verlängerter Reaktionszeit oder Lagerung des Produktes nicht wesentlich weiter absinkt.

Die Auswahl der eingesetzten Diisocyanatverbindungen ist durch den Verfahrensanspruch bereits festgelegt. Für die Vollblockierung kann jedes Diisocyanat herangezogen werden, soferne seine NCO-Gruppen einer Blockierung, sowie der Entblockierung bei Temperaturen zwischen 120 und 200°C, zugänglich sind.

Als Diisocyanate für die Reaktion mit dem vollblockierten Diisocyanat müssen zur Erzielung einheitlicher Produkte Verbindungen eingesetzt werden, deren NCO-Grupen unterschiedliche Reaktivität aufweisen. Beispiele für handelsübliche Produkte dieser Art sind das Isophorondiisocyanat oder das 2,4-Toluylendiisocyanat. Beim Einsatz technischer Isomerengemische, wie sie bei vielen Handelsprodukten des Toluylendiisocyanats vorliegen (80 % 2,4- und 20 % 2,6-TDI) ist mit Abweichungen zu rechnen, die jedoch für die weitere Verwendung meist ohne wesentliche Bedeutung sind.

Als Blockierungsmittel werden die aus der Literatur bekannten, ein reaktives Wasserstoffatom aufweisenden, Verbindungen eingesetzt (siehe z.B. "Methoden der Organischen Chemie", (HOUBEN-WEYL), Bd. 14/2, Seiten 61 - 70, G. Thieme-Verlag, Stuttgart 1963). Definitionsgemäß müssen die Blockierungsmittel bei einer für die Praxis brauchbaren Temperatur unter intermediärer Rückbildung der Isocyanatgruppe abgespalten werden.

Beispiele für Blockierungsmittel sind

Monohydroxylverbindungen, wie Alkanole, Glykolmonoether, Hydroxyalkylacrylate, Dialkylalkanolamine, Ketoxime,

Lactame, wie epsilon-Caprolactam oder delta-Valerolactam,

CH-aktive Verbindungen wie Acetylaceton, Acetessigester oder Malonesterderivate,

aliphatische Amine, wie 2-Ethylhexylamin, Dibutylamin, Dimethyl- oder Diethylaminopropylamin.

Selbstverständlich können auch Mischungen verschiedener Blockierungsmittel eingesetzt werden. Bei Verwendung geeigneter Aminoalkohole, wie der genannten Dialkylalkanolamine, oder von prim.-tert. Aminen wird durch das Blockierungsmittel gleichzeitig eine protonierbare, bei der Härtung sich abspaltende Gruppe eingeführt.

Bei der Herstellung der erfindungsgemäßen Isocyanatverbindungen wird das Diisocyanat bei 30 bis 50°C mit dem Blockierungsmittel in einem solchen Mengenverhältnis umgesetzt, daß beide NCO-Gruppen blockiert werden. Das vollblockierte Diisocyanat wird dann mit einer äquimolaren Menge eines Diisocyanates, welches unterschiedlich reaktive NCO-Gruppen aufweist, bei 70 bis 120°C zum Monoisocyanat umgesetzt. Gegebenenfalls kann die Reaktion durch basische Katalysatoren, wie Triethylamin oder auch ein basisches Blockierungsmittel, beschleunigt werden. Vorteilhafterweise wird die Zugabe des Diisocyanats bei ca. 70°C begonnen und die Temperatur nach Maßgabe des Reaktionsablaufes gesteigert. Bei der angewandten Maximaltemperatur darf weder eine Reaktion der zweiten NCO-Gruppe noch eine Abspaltung des Blokkierungsmittels eintreten. Unter den gegebenen Bedingungen kommt die Reaktion bei halbseitiger Umsetzung des Diisocyanats zum Stillstand.

Die erfindungsgemäßen Isocyanatverbindungen dienen als Härtungskomponenten für NCO-reaktive Harzsysteme, die bei erhöhter Temperatur vernetzt werden. Insbesonders können sie durch Reaktion ihrer freien NCO-Gruppe mit einer NCO-reaktiven Gruppe des Bindemittels zur Herstellung selbstvernetzender Systeme dienen.

Die folgenden Beispiele erläutern die Erfindung. Alle Angaben in Teilen oder Prozenten beziehen sich, soferne nichts anderes angegeben ist, auf Gewichtseinheiten. Der NCO-Wert gibt die Menge der freien Isocyanatgruppen in Gew.-% an.

Beispiele 1 - 8

Das für die Vollblockierung vorgesehene Diisocyanat (I) (1 Mol) wird in einem geeigneten Reaktionsgefäß vorgelegt und das Blockierungsmittel (2 Mol) bei 30 bis 40°C unter Kühlung kontinuierlich zugegeben. Anschließend wird die Temperatur weiter bis zum vollständigen Umsatz aller NCO-Gruppen gehalten. Nach Erwärmen auf 70°C wird, gegebenenfalls nach Zusatz eines Katalysators, das für die weitere Reaktion vorgesehene Diisocyanat (II) (1 Mol) langsam zugegeben. Die Temperatur wird dabei langsam auf die vorgesehene Maximaltemperatur gesteigert. Wenn der theoretische NCO-Wert für das Monoisocyanat erreicht ist, wird die Temperatur noch 15 Minuten gehalten. Das Produkt kann unmittelbar weiterverarbeitet oder bei Raumtemperatur gelagert werden.

Beim Einsatz von Isocyanaten mit unterschiedlich reaktiven NCO-Gruppen als Diisocyanat (I) und als Diisocyanat (II) kann die Reaktion auch in der Weise erfolgen, daß das Blockierungsmittel (2 Mol) im Reaktionsgefäß vorgelegt wird, die erste Hälfte (1 Mol) des Diisocyanats bei 30 bis 40°C zugegeben und bis zur Vollblockierung reagiert wird. Anschließend wird unter Temperaturerhöhung die zweite Hälfte (1 Mol) des Diisocyanates zugegeben und reagiert.

Die Mengenverhältnisse, Reaktionsbedingungen und Kennwerte für die Beispiele sind in der folgenden Tabelle zusammengefaßt, wobei folgende Abkürzungen benutzt werden:

| | |
|---|---|
| TDI/80 | Toluylendiisocyanat (handelsübliches Isomerengemisch; 80 % 2,4-; 20 % 2,6-TDI) |
| 2,4-TDI | 2,4-Toluylendiisocyanat (techn.) |
| TMHMDI | Trimethylhexamethylendiisocyanat |
| IPDI | Isophorondiisocyanat |
| EH | 2-Ethylhexanol |
| BG | Monoethylenglykolmonobutylether |
| DEOLA | Diethylethanolamin |
| BOX | Butanonoxim |
| DEAPA | Diethylaminopropylamin |
| AC | Acetylaceton |
| TEA | Triethylamin |
| CL | epsilon-Caprolactam |
| DMBA | Dimethylbenzylamin |

Tabelle 1

| Beispiel | Diisocyanat (I) Tle (jeweils 1 Mol) | Blockierungsmittel Tle (Mol) | Diisocyanat (II) Tle (jeweils 1 Mol) | Katalysator Gew.-% | Max.Reaktionstemp. °C | Molmasse | NCO theor. | Wert gef. |
|---|---|---|---|---|---|---|---|---|
| 1 | 174 TDI/80 | 260 (2,0) EH | 222 IPDI | 0,5 TEA | 120 | 656 | 6,4 | 6,35 |
| 2 | 210 TMHMDI | 177 (1,5) BG<br>59 (0,5) DEOLA | 174 2,4-TDI | -- | 105 | 620 | 6,8 | 6,7 |
| 3 | 222 IPDI | 174 (2,0) BOX | 222 IPDI | -- | 110 | 618 | 6,8 | 6,8 |
| 4 | 174 TDI/80 | 234 (1,8) EH<br>26 (0,2) DEAPA | 174 2,4-TDI | -- | 105 | 608 | 6,9 | 6,9 |
| 5 | 210 TMHMDI | 118 (1,0) BG<br>113 (1,0) CL | 222 IPDI | 0,8 TEA | 120 | 663 | 6,3 | 6,2 |
| 6 | 174 TDI/80 | 130 (1,0) EH<br>117 (1,0) DEOLA | 222 IPDI | -- | 110 | 643 | 6,5 | 6,5 |
| 7 | 174 TDI/80 | 200 (2,0) AC | 174 2,4-TDI | 0,5 DMBA | 120 | 548 | 7,7 | 7,6 |
| 8 | 210 TMHMDI | 177 (1,5) BG<br>59 (0,5) DEOLA | 174 TDI/80 | -- | 110 | 620 | 6,1 | 5,4 |

## Patentansprüche

### Patentansprüche für folgenden Vertragsstaaten : BE, DE, FR, GB, IT, NL, SE

1. Verfahren zur Herstellung von diblockierten Triisocyanatverbindungen, dadurch gekennzeichnet, daß man ein Mol eines vollblockierten Diisocyanates mit einem Mol eines nichtblockierten Diisocyanates, dessen NCO-Gruppen überwiegend unterschiedliche Reaktivität aufweisen, bei 70 bis 120°C, gegebenenfalls in Gegenwart eines Katalysators, bis zu einem der Monoisocyanatverbindung entsprechenden NCO-Wert reagiert, wobei die maximale Reaktionstemperatur so gewählt wird, daß noch keine wesentliche Reaktion der freien NCO-Gruppen und/oder noch keine wesentliche Abspaltung des Blockierungsmittels erfolgt.

2. Verfahren nach Anspruch 1, dadurch gekennzeichnet, daß man die Zugabe des nichtblockierten Diisocyanates bei steigender Temperatur, beginnend bei 70°C, durchführt.

3. Verfahren nach den Ansprüchen 1 und 2, dadurch gekennzeichnet, daß man als nichtblockiertes Diisocyanat ein technisches 2,4-Toluylendiisocyanat mit bis zu 20 Gew.-% 2,6-Isomerenanteil einsetzt.

4. Diblockierte Triisocyanate der allgemeinen Formel

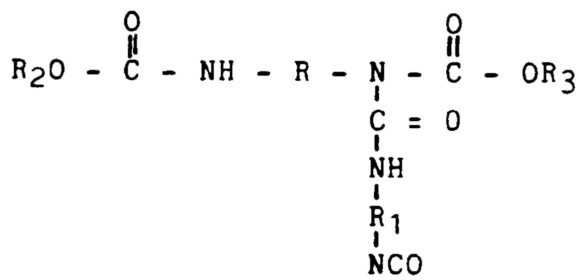

wobei

R ein von einem Diisocyanat stammender aliphatischer, cycloaliphatischer oder aromatischer Rest,

$R_1$ ein von einem Diisocyanat, dessen NCO-Gruppen überwiegend verschiedene Reaktivität aufweisen, stammender aliphatischer, cycloaliphatischer oder aromatischer Rest ist

und

$R_2$ und $R_3$ gleiche oder verschiedene Reste von NCO-Blokkierungsmitteln darstellen.

5. Diblockierte Triisocyanate nach Anspruch 4, dadurch gekennzeichnet, daß der Rest R von Diisocyanaten mit unterschiedlich reaktiven NCO-Gruppen stammt.

6. Verwendung der diblockierten Triisocyanate nach den Ansprüchen 4 und 5 als Vernetzungskomponenten für Hydroxylgruppen und/oder primäre und/oder sekundäre Aminogruppen aufweisende Lackbindemittel, vorzugsweise zur Einführung blockierter Urethanvernetzungsgruppen in solche Lackbindemittel.

### Patentanspruch für folgenden Vettragsstaat : ES

1. Verfahren zur Herstellung von diblockierten Triisocyanatverbindungen, dadurch gekennzeichnet, daß man ein Mol eines vollblockierten Diisocyanates mit einem Mol eines nichtblockierten Diisocyanates, dessen NCO-Gruppen überwiegend unterschiedliche Reaktivität aufweisen, bei 70 bis 120°C, gegebenenfalls in Gegenwart eines Katalysators, bis zu einem der Monoisocyanatverbindung entsprechenden NCO-Wert reagiert, wobei die maximale Reaktionstemperatur so gewählt wird, daß noch keine wesentliche Reaktion der freien NCO-Gruppe und/oder noch keine wesentliche Abspaltung des Blockierungsmittels erfolgt.

2. Verfahren nach Anspruch 1, dadurch gekennzeichnet, daß man die Zugabe des nichtblockierten Diisocyanates bei steigender Temperatur, beginnend bei 70°C, durchführt.

3. Verfahren nach den Ansprüchen 1 und 2, dadurch gekennzeichnet, daß man als nichtblockiertes Diisocyanat ein technisches 2,4-Toluylendiisocyanat mit bis zu 20 Gew.-% 2,6-Isomerenanteil einsetzt.

4. Verwendung der nach den Ansprüchen 1 bis 3 hergestellten diblockierten Triisocyanate als Vernetzungskomponenten für Hydroxylgruppen und/oder primäre und/oder sekundäre Aminogruppen aufweisende Lackbindemittel, vorzugsweise zur Einführung blockierter Urethanvernetzungsgruppen in solche Lackbindemittel.

## Claims

**Claims for the following Contracting States : BE, DE, FR, GB, IT, NL, SE**

1. Process for the preparation of doubly blocked triisocyanate compounds, characterized in that one mol of a fully blocked diisocyanate is reacted, at 70 to 120°C and in the presence or absence of a catalyst, with one mol of a non-blocked diisocyanate whose NCO groups have predominantly different reactivities until an NCO value is reached which corresponds to the monoisocyanate compound, the maximum reaction temperature being chosen such that no significant reaction of the free NCO group and/or no significant cleavage of the blocking agent occurs.

2. Process according to Claim 1, characterized in that the addition of the non-blocked diisocyanate is carried out at increasing temperature, starting at 70°C.

3. Process according to Claims 1 and 2, characterized in that technical 2,4-toluylene diisocyanate comprising up to 20% by weight of the 2,6-isomer is used as the non-blocked diisocyanate.

4. Doubly blocked triisocyanates of the general formula

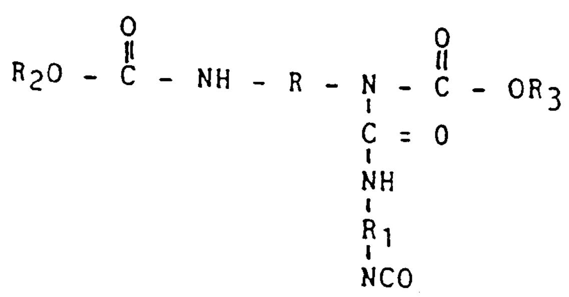

in which

R is an aliphatic, cycloaliphatic or aromatic radical derived from a diisocyanate,

$R_1$ is an aliphatic, cycloaliphatic or aromatic radical derived from a diisocyanate whose NCO groups have predominantly different reactivities and

$R_2$ and $R_3$ represent identical or different radicals of NCO blocking agents.

5. Doubly blocked triisocyanates according to Claim 4, characterized in that the radical R is derived from diisocyanates with NCO groups of different reactivities.

6. Use of the doubly blocked triisocyanates according to Claims 4 and 5 as crosslinking components for paint binders comprising hydroxyl groups and/or primary and/or secondary amino groups, preferably for introducing blocked urethane crosslinking groups into such paint binders.

**Claims for the following Contracting State : ES**

1. Process for the preparation of doubly blocked triisocyanate compounds, characterized in that one mol of a fully blocked diisocyanate is reacted, at 70 to 120°C and in the presence or absence of a catalyst, with one mol of a non-blocked diisocyanate whose NCO groups have predominantly different reactivities until an NCO value is reached which corresponds to the monoisocyanate compound, the maximum reaction temperature being chosen such that no significant reaction of the free NCO group and/or no significant

cleavage of the blocking agent occurs.

2. Process according to Claim 1, characterized in that the addition of the non-blocked diisocyanate is carried out at increasing temperature, starting at 70°C.

3. Process according to Claims 1 and 2, characterized in that technical 2,4-toluylene diisocyanate comprising up to 20% by weight of the 2,6-isomer is used as the non-blocked diisocyanate.

4. Use of the doubly blocked triisocyanates prepared according to Claims 1 to 3 as crosslinking components for paint binders comprising hydroxyl groups and/or primary and/or secondary amino groups, preferably for introducing blocked urethane crosslinking groups into such paint binders.

## Revendications

## Revendications pour les Etats contractants suivants : BE, DE, FR, GB, IT, NL, SE

1. Procédé de préparation de triisocyanates dibloqués, caractérisé en ce qu'on fait réagir une mole d'un diisocyanate complètement bloqué avec une mole d'un diisocyanate non bloqué dont les groupes NCO présentent des réactivités essentiellement différentes, à une température de 70 à 120°C, éventuellement en présence d'un catalyseur, jusqu'à un indice de NCO correspondant au monoisocyanate, la température de réaction maximale étant choisie de façon à ce qu'il ne se produise pas encore de réaction importante des groupes NCO libres et/ou à ce qu'il n'y ait pas encore de séparation importante de l'agent de blocage.

2. Procédé selon la revendication 1, caractérisé en ce qu'on effectue l'addition du diisocyanate non bloqué à une température croissante, à partir de 70°C.

3. Procédé selon les revendications 1 et 2, caractérisé en ce qu'on utilise, comme diisocyanate non bloqué, un diisocyanate de 2,4-tolylène technique ayant une proportion d'isomère 2,6 allant jusqu'à 20 % en poids.

4. Triisocyanates dibloqués de formule générale

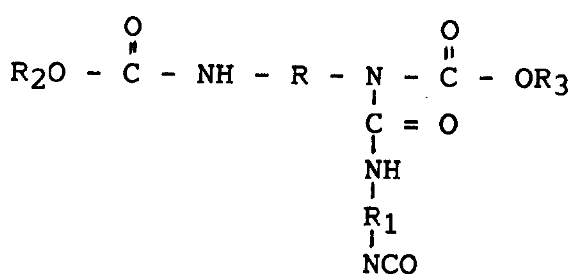

dans laquelle
R représente un reste aliphatique, cycloaliphatique ou aromatique issu d'un diisocyanate,
$R_1$ est un reste aliphatique, cycloaliphatique ou aromatique issu d'un diisocyanate dont les groupes NCO présentent des réactivités essentiellement différentes, et
$R_2$ et $R_3$ représentent des restes identiques ou différents d'agents de blocage de NCO.

5. Triisocyanates dibloqués selon la revendication 4, caractérisés en ce que le reste R est issu de diisocyanates ayant des groupes NCO de réactivités différentes.

6. Utilisation des triisocyanates dibloqués selon les revendications 4 et 5 comme constituants de réticulation pour des liants de vernis qui présentent des groupes hydroxyle et/ou des groupes amino primaires et/ou secondaires, de préférence pour l'introduction de groupes de réticulation d'uréthanne bloqués dans de tels liants de vernis.

**Revendications pour l'Etat contractant suivant : ES**

1. Procédé de préparation de triisocyanates dibloqués, caractérisé en ce qu'on fait réagir une mole d'un diisocyanate complètement bloqué avec une mole d'un diisocyanate non bloqué dont les groupes NCO présentent des réactivités essentiellement différentes, à une température de 70 à 120°C, éventuellement en présence d'un catalyseur, jusqu'à un indice de NCO correspondant au monoisocyanate, la température de réaction maximale étant choisie de façon à ce qu'il ne se produise pas encore de réaction importante des groupes NCO libres et/ou à ce qu'il n'y ait pas encore de séparation importante de l'agent de blocage.

2. Procédé selon la revendication 1, caractérisé en ce qu'on effectue l'addition du diisocyanate non bloqué à une température croissante, à partir de 70°C.

3. Procédé selon les revendications 1 et 2, caractérisé en ce qu'on utilise, comme diisocyanate non bloqué, un diisocyanate de 2,4-tolylène technique ayant une proportion d'isomère 2,6 allant jusqu'à 20 % en poids.

4. Utilisation des triisocyanates dibloqués préparés selon les revendications 1 à 3 comme constituants de réticulation pour des liants de vernis qui présentent des groupes hydroxyle et/ou des groupes amino primaires et/ou secondaires, de préférence pour l'introduction de groupes de réticulation d'uréthanne bloqués dans de tels liants de vernis.